(19) Europäisches Patentamt
European Patent Office
Office européen des brevets

(11) Publication number: 0 338 532 A2

# (12) EUROPEAN PATENT APPLICATION

(21) Application number: 89107027.8

(51) Int. Cl.⁴: A61K 31/38 , A61K 31/54

(22) Date of filing: 19.04.89

(30) Priority: 20.04.88 US 183858
12.09.88 US 243524

(43) Date of publication of application:
25.10.89 Bulletin 89/43

(84) Designated Contracting States:
AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Bitonti, Alan J.
7854 Carraway Court
Maineville Ohio 45039(US)
Inventor: McCann, Peter P.
3782 Cooper Road
Cincinnati Ohio 45241(US)
Inventor: Sjoerdsma, Albert
5475 Waring Drive
Cincinnati Ohio 45243(US)

(74) Representative: Vossius & Partner
Siebertstrasse 4 P.O. Box 86 07 67
D-8000 München 86(DE)

## (54) N-alkylamino derivates of aromatic, tricyclic compounds in the treatment of drug-resistant protozoal infections.

(57) Drug-resistant protozoal infection, particularly, drug-resistant malarial infection in humans, can be effectively treated with standard antiprotozoal agents if administered in conjunction with a tricyclic N-aminoalkyl iminodibenzyl, dibenzylcycloheptane and cycloheptene, phenothiazine, dibenzoxazepine, dibenzoxepine, or thioxanthene derivative.

EP 0 338 532 A2

Xerox Copy Centre

# N-ALKYLAMINO DERIVATIVES OF AROMATIC, TRICYCLIC COMPOUNDS IN THE TREATMENT OF DRUG-RESISTANT PROTOZOAL INFECTIONS

This invention relates to the use of certain N-(aminoalkyl) derivatives of iminodibenzyl, dibenzylcycloheptane and cycloheptene, phenothiazine, dibenzoxazepine, dibenzoxepine, and thioxanthene in the treatment of drug-resistant malaria and in the treatment of other drug-resistant protozoal infections.

Malaria remains a significant health threat to humans despite massive international attempts to eradicate the disease. Over 200 million people are said to have malaria and over one million deaths per year are associated with malaria in Africa alone. In many of the endemic areas, local supply of food is quite limited, a problem which is greatly aggravated by the presence of protozoal infections in cattle and other farm animals.

Malaria is a disease of warm blooded animals caused by infection with a parasite of the genus Plasmodium. Four species, P. *vivax*, P. *falciparum*, P. *malariae*, and P. *ovale*, are known to infect humans. The parasite is transmitted to humans by the bite of *Anopheles* mosquitoes. Subsequent to mosquito bite, the parasite rapidly invades the blood cells of the victim and after a incubation period, generally lasting about 10 to 14 days, symptoms, consisting of chills, fever, headache, muscle pains, spenomegaly, and anemia, appear. This incubation period may be prolonged for many weeks and onset can be quite insidious. Red blood cells are at first altered and later destroyed by the infection.

Drug therapy utilizing quinine, chloroquine, amodiaquine, primaquine, and other agents has been the mainstay of therapy against malaria. However, drug-resistant strains of protozoa have developed and in some cases strains are resistant to many or all of the current therapeutic agents. In particular, P. *falciparum* malaria is quite prone to exhibit single and even multiple drug-resistance. While new agents are continually developed and introduced, resistance to such new agents also quickly develops. For example mefloquine-resistant malaria was reported even before mefloquine licensure was completed. There is, thus, an urgent need for antimalarial and antiprotozoal agents which can be used in the treatment of drug-resistant malaria and other protozoal diseases.

Recently it was reported that imipramine and amitryptyline suppress P. *falciparum* growth, possibly by virtue of the ability of these agents to interfere with riboflavin metabolism. While scientifically interesting, the practical use of imipramine and amitryptyline in the treatment of malaria would seem unlikely because of the lethal concentrations required to produce the anti-malarial effect in humans. While not practically useful in the treatment of non drug-resistant malaria, applicants have now discovered that these and other such tricyclic antidepressant and antipsychotic compounds, when admininstered in subtoxic doses in conjunction with standard antimalarial agents, are highly effective in treating drug-resistant malarial and other drug-resistant protozoal infections and are useful in the prophylaxis of drug-resistant malarial and other protozoal infections.

Applicants have found that certain N-(aminoalkyl) derivatives of iminodibenzyl, dibenzylcycloheptane and cycloheptene, phenothiazine, dibenzoxazepine, dibenzoxepine, and thioxanthene derivatives of formula I

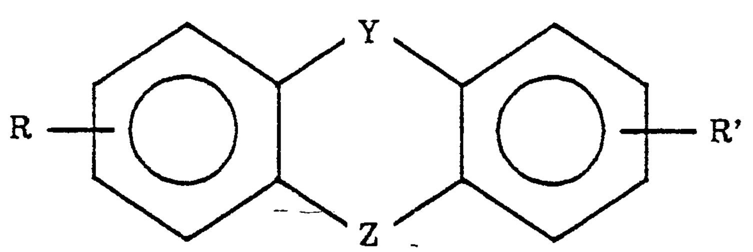

(I)

wherein

Z is a divalent thio, 1,2-ethanediyl, 1,2-ethenediyl, or methyleneoxy radical;

Y is a divalent radical of one of the formulae:

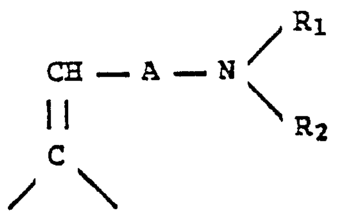 , 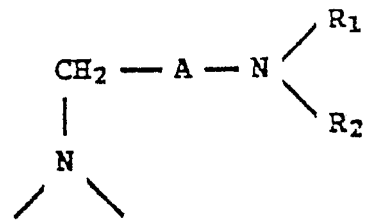 ,

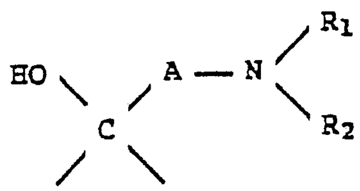 , 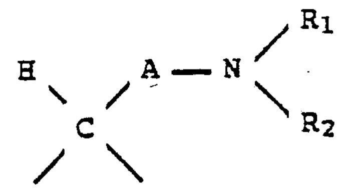 ;

A is a alkylene group of from 1 to 4 carbon atoms;
$R_1$ and $R_2$ are each, independently, a hydrogen or an alkyl group of from 1 to 4 carbon atoms or
$R_1$ and $R_2$ taken together with the nitrogen atom to which they are attached can form a N-piperazino- optionally substituted with a methyl, ethyl, or 2-hydroxyethyl group, N-piperidino-, N-morpholino-, or N-pyrrolidino radical;
R and $R'$ are each, independently, a hydrogen, hydroxy, bromo, chloro, iodo, or trifluoromethyl group or an alkyl or alkoxy group of from 1 to 4 carbon atoms;
or a pharmaceutically acceptable salt thereof when administered conjunctively with an antiprotozoal agent are useful in the treatment of individuals suffering from and prevention of drug-resistant malaria and other drug-resistant protozoal infections.

As used herein the term "alkyl group of from 1 to 4 carbon atoms" encompasses the straight, branched, and cyclic alkyl groups such as methyl, ethyl, n-propyl, isopropyl, cyclopropyl, n-butyl, isobutyl, tert-butyl, sec-butyl, cyclopropylmethyl, cyclobutyl, n-pentyl, isopentyl, sec-pentyl, cyclopentyl, cyclobutylmethyl, cyclopropylethyl, and methylcyclopropylmethyl. The term "alkoxy group of from 1 to 4 carbon atoms" means those alkoxy groups wherein the alkyl portion is an alkyl group of 1 to 4 carbon atoms as defined.

As used herein, the term "alkylene group of from 1 to 4 carbon atoms" means those saturated straight and branched divalent hydrocarbon groups wherein the open valancies are on other than adjacent carbon atoms. Examples of such alkylene groups are 1,2-ethanediyl, 1,3-propanediyl, 1-methyl-1,2-ethanediyl, 1,4-butanediyl, and 2-methyl-1,3-propanediyl.

The iminodibenzyl, dibenzylcycloheptane and cycloheptene, phenothiazine, dibenzoxazepine, dibenzoxepine, and thioxanthene derivatives of this invention are well known compounds, and are members of the class of antidepressant compounds referred to as the tricyclic antidepressant compounds. These compounds are widely available or can be readily prepared by those of ordinary skill in the art of preparing benzocyclic compounds.

Examples of tricyclic antidepressant and antipsycotic compounds encompassed within the method of this invention are:
Chlorpromazine hydrochloride;
Promazine hydrochloride;
Triflupromazine hydrochloride;
Fluphenazine hydrochloride;
Perphenazine;
Prochlorperazine maleate;
Trifluoperazine hydrochloride;
Chlorprothixine;
Cidoxepin hydrochloride;
Clomacran phosphate;
Clopenthixol;
Dimeprozan;
Doxepin hydrochloride;

Trimerprazine;
Imipramine;
Desipramine;
Amitriptyline;
Trimipramine;
Nortriptyline;
2-Hydroxyimipramine;
2-Hydroxydesipramine; and
Protriptyline.

The preferred compounds of formula 1 to be used in the method of this invention are those compounds wherein at least one of $R_1$ and $R_2$ are an alkyl group of from 1 to 5 carbon atoms, as well as those compounds of formula 1 wherein R and $R'$ are each, independently, a hydrogen. More preferred are those compounds wherein one of $R_1$ and $R_2$ are a methyl group and the other os a hydrogen or a methyl group as well as those compounds having from one to three carbon atoms between the tricyclic ring and the nitrogen atom to which the $R_1$ and $R_2$ groups are attached. Even more preferred are those iminodibenzyl, dibenzocycloheptane, and dibenzocycloheptene compounds of formula 1 wherein there are at least two, and preferably three, carbon atoms between the tricyclic ring and the nitrogen atom to which the $R_1$ and $R_2$ groups are attached as well as those compounds wherein at least one of $R_1$ and $R_2$ is a methyl group and the other is a hydrogen or a methyl group. Most preferred are those iminodibenzyl, dibenzylcycloheptane, and dibenzylcycloheptene compounds wherein there are three carbon atoms between the tricyclic ring and the nitrogen atom to which the $R_1$ and $R_2$ groups are attached, wherein R and $R'$ are each a hydrogen, and wherein one of $R_1$ and $R_2$ is a methyl group and the other is a hydrogen or wherein both of $R_1$ and $R_2$ are methyl groups. The compounds imipramine, desipramine, amitriptyline, and protriptyline are examples of the most preferred compounds with desipramine being preferred above all other compounds for use in the methods of this invention. Prophylactic therapy of protozoal infections such as malarial infections is normally accomplished by administration of the antiprotozoal agent in a once or twice a week dosage form. When the compounds of formula 1 are administered in conjunction with an antiprotozoal agent for protozoal infection prophylaxis, it is preferable to employ a compound of formula 1 which has a comparable blood plasma half-life or which can be administered in a sustained dosage form is desirable. For example, chloroquine is administered once a week for prophylaxis of malarial infections. When selecting a formula 1 compound for conjunctive administration with chloroquine for drug-resistant malaria prophylaxis, a formula 1 compound with suitably long plasma half-life such as protriptyline or which can be administered in a suitable week long dosage form is preferred.

The compounds of this invention are useful both in the free base form and in the form of acid addition salts. The acid addition salts are simply a more convenient form for use and, in practice, use of the salt amounts to use of the free base. The expression "pharmaceutically acceptable acid addition salts" is intended to apply to any non-toxic organic or inorganic acid addition salts of the base compounds of formula 1. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case the salt separates directly or can be obtained by concentration of the solution.

It will be readily apparent to those skilled in the art that certain of the compounds of this invention may exhibit geometric isomerism. For example, those compounds of formula 1 wherein the Y group contains a carbon-carbon double bond may have geometric isomers such as where the tricyclic core ring system is substituted by dissimilar R and $R'$ groups or where Z is a methyleneoxy radical. Moreover many of the compounds of structure 1 may have stereochemical isomers. For example, the carbon atom of the group which is part of the tricyclic ring system in those compounds wherein the Y is $=C(OH)AN(R_1)(R_2)$ or $=C(H)AN(R_1)(R_2)$ is achiral and these compounds can exist as either of the stereoisomers or as a mixture of the isomers. Unless specifically indicated, the individual isomers, geometrical or sterochemical, and their mixtures are intended.

As used herein, the term "protozoal infections" means those protozoal iodium (including such species as vivax, malariae, ovale, falciparum, knowlesi, berghei, vinckei, chabaudi, gallinaceum, and lophurae), Leishmania (including such species as donovani, tropica, braziliensis, and mexicana), Trypanosoma

(including such species as cruzi), Babesiosis, and Theileria species. The most important use contemplated for the present invention is its use in the treatment of drug-resistant Plasmodium infections, especially malarial infections of drug-resistant strains of P. *falciparum*, in humans.

The term "drug-resistant protozoal infections" means those protozoal infections in which the infecting strain of protozoa is either completely or substantially refractory to therapy or prophylaxis with existing antiprotozoal agents or combinations of antiprotozoal agents. The term "drug-resistant malaria" means a malarial infection, particularly of malaria resulting from infection by P. *falciparum* in humans, which are substantially not responsive to treatment or prophylaxis with existing therapeutic and prophylactic agents such as quinine, chloroquine, amodiaquine, primaquine, or mefloquine.

The antiprotozoal agents used in the combination therapy or prophylaxis of this invention include any therapeutic or prophylatic agent used in the treatment or prophylaxis of non drug-resistant protozoal infections. As used herein, the terms "antiprotozoal agent" and "antimalarial agent" specifically do not include the compounds of formula 1. Examples of antiprotozoal agents used in the treatment or prophylaxis of malarial infections are various quinoline derivatives such as quinine, chloroquine, primaquine, sulfadoxine, mefloquine, and pyrimethamine. Examples of antiprotozoal agents used in the treatment or prophylaxis of Leishmaniasis infections are various pentavalent antimony compounds such as sodium stibogluconate, amphotericin B, and pentamidine isethionate. Examples of antiprotozoal agents used in the treatment or prophylaxis of Trypanosomiasis infections are nifurtimox, suramin, melarsoprol, alpha-difluoromethylornithine (DFMO), and pentamidine. Various salts of these agents may also be employed and combinations of these various agents are routinely utilized.

The term "conjunctive" when to describe the treatment or prophylaxis of this invention, contemplates the administration of a formula 1 compound immediately prior to, concomitantly with, or subsequent to treatment with the antiprotozoal agent or agents. Applicants contemplate that the tricyclic antidepressant or antipsychotic compounds of this invention may be formulated into a single dosage form together with the antiprotozoal agent; however, such a combintaion dosage form is not required in order to practice the method of this invention, and no advantage results from use of such a combination product. Rather, because the antiprotozoal agents and tricyclic antidepresant and antipsychotic compounds are widely availabe in seperate dosage forms, applicants expect that patients will be treated using such available, seperate dosage forms although a combination dosage form is contemplated especially for the prophylaxis of drug-resistant protozoal infections. Typically, treatment of a patient infected with a drug-resistant protozoa, requires doses of the antiprotozoal agent or agents many times the normal dosage, and such therapy is heroic in nature, i.e., in an effert to save the life of the patient, doses of antiprotozoal agents normally regarded as "overdosages" are used and symptomatic relief of overdosage symptoms are tended to on an individual basis. While the conjunctive therapy and prophylaxis of this invention will provide for use of less antiprotozoal agent than would be possible in the absence of the formula 1 compound, applicants contemplate that the dose of antiprotozoal agent employed in the method of this invention will be essentially that dose which would be employed in the absence of the formula 1 compound when used in the treatment or prophylaxis of a non drug-resistant protozoal infection. Rather than decreasing the dose of antiprotozoal agent required, the conjunctive therapy and prophylaxis of this invention will provide for treatment or prophylaxis of protozoal infections which would otherwise not be adequately treated or prevented in the absence of a formula 1 compound. In fact, it is the very gist of applicants' discovery that protozoal infections which are not responsive to therapy or prophylaxis with standard antiprotozoal agents, become sensitive to such therapy or prophylaxis when administered in conjunction with a compound of formula 1.

The preferred route of administration is oral administration. For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

Pharmaceutical compositions of many of the compounds of formula 1 such as imipramine, desipramine, chloropromazine, and amitriptyline and of the antiprotozoal agents are widely available and can be used in the practice of this invention. Commonly available dosage forms of the tricyclic antidepressant and antipsychotic compounds of this invention contain from 5 to 250 mg and are to be administered to a human patient from 1 to 3 or 4 times daily, as required. Such dosage forms and frequency of administration are suitable for use in practising the method of this invention. Pharmaceutical compositions of antimalarial agents such as chloroquine and compositions of other antiprotozoal agents are also widely available and can be used in the practice of the method of this invention. Combination dosage forms, those containing both a formula 1 compound and an antiprotozoal agent are also specifically contemplated for use in the methods of this inventions particularly in the prophylactic methods of this invention.

The ability of the compounds of formula 1 to treat drug-resistant protozoal infections can be demonstrated by following the incorporation of [$^3$H]-hypoxanthine (Desjardins, *et al*, (1979) Antimicrobial Agents Chemotherapy 16, 710-718) into drug-resistant Plasmodium *falciparum* using standard techniques. P. *falciparum* (clone D6, chloroquine-sensitive; strain FCR3, chloroquine-resistant; and clone W2, multidrug-resistant) was grown *in vitro* by the method of Trager and Jensen, (1976) Science 193, 673-675.

Figure 1 illustrates the effect of desipramine on the inhibition of parasite growth. The degree of sensitivity or resistance of the parasites to chloroquine was found to be inversely related to the sensitivity of the parasites to desipramine.

Figure 2 illustrates the synergism of desipramine and chloroquine against chloroquine-resistant P. *falciparum* (FCR3). Isobologram analysis was carried out according to the method of Martin, et al., (1987) Science 235, 899-901 and Berenbaum, (1978) Journal of Infectious Diseases 137, 122-130. Drug synergy in this test is demonstrated when a test curve (solid line, desipramine + chloroquine) falls to the left of the theoretical additivity line as is the case with the desipramine + chloroquine curve. The X-axis is the $IC_{50}$ - (concentration of drug which inhibits hypoxanthine incorporation by 50%) of chloroquine in the absence or presence of desipramine while the Y-axis is the $IC_{50}$ for desipramine in the absence or presence of chloroquine. The more highly synergistic a combination is, the closer to the origin the points will fall. The combination of desipramine and chloroquine is shown to be highly synergistic.

Figure 3 illustrates the ability of desipramine to enhance the potency of chloroquine against P. *falciparum* (FCR3). Dose-response curves and $IC_{50}$'s for chloroquine were determined either in the absence of desipramine or in the presence of a fixed concentration of desipramine ranging from 50 ng

desipramine/ml up to 500 ng desipramine/ml. As the concentration of desipramine was increased the dose-response curves shifted to the left, thus indicating that desipramine enhanced the potency of chloroquine. Clinically achievable plasma concentrations of desipramine in depressed patients are in the range of 50 - 200 ng/ml. Desipramine (500 ng/ml) lowered the $IC_{50}$ and $IC_{90}$ for chloroquine by 10-fold. An $IC_{50}$ for a chloroquine-sensitive P. *falciparum* strain would be approximately 5 ng/ml. Therefore in the presence of clinically relevant concentrations of desipramine, the chloroquine-resistant P. *falciparum* strain is as sensitive to chloroquine as is the "sensitive" strain.

Figure 4 illustrates the ability of desipramine to enhance the potency of chloroquine against P. *falciparum* (W2). The experiment is identical to that of Figure 3 but a different strain of P. *falciparum* was used. The W2 strain is more sensitive to desipramine. The $IC_{50}$ and $IC_{90}$ for chloroquine are lowered by a factor of approximately 10. The concentrations of desipramine used have only slight effects of P. *falciparum* when desipramine was added alone.

Figure 5 illustrates that desipramine does not change the response of the chloroquine-susceptible clone D6 of *P. falciparum* to chloroquine in an experiment similar to those in Figures 3 and 4.

Figure 6 illustrates the synergism of chloropromazine and chloroquine. The anti-psycotic, chloropromazine, was substituted for desipramine in an experiment similar to that in Figure 2.

Figure 7. Desipramine increases the accumulation of chloroquine by drug-resistant *P. falciparum* but ot by drug-susceptible parasites. Uptake of [3H]-chloroquine was followed in clone D6 (chloroquine susceptible, •) and in clone W2 (multidrug-resistant, o) in the absence or presence of desipramine.

The following example is intended to illustrate, but not to limit in any way, the compositions useful in the methods of this invention.

## Example

Tablets

Tablets were prepared in the usual manner each having the composition:

| | |
|---|---|
| Chloroquine phosphate | 250 mg |
| Desipramine HCl | 75 mg |
| Dibasic calcium phosphate | 50 mg |
| Lactose | 250 mg |
| Magnesium stearate | 10 mg |
| Starch | 100 mg |

## Claims

1. The use of a tricyclic compound of the formula I

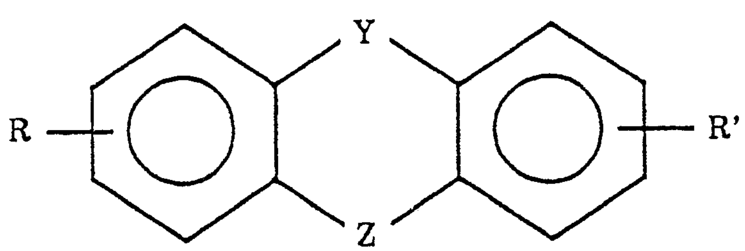

(I)

wherein
Z is a divalent thio, 1,2-ethanediyl, 1,2-ethenediyl, or methyleneoxy radical;

Y is a divalent radical of one of the formulae:

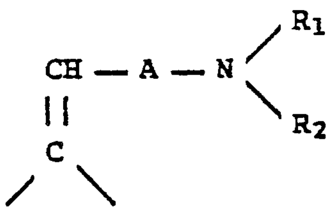 , 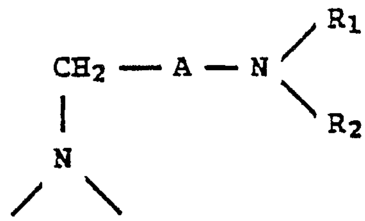 ,

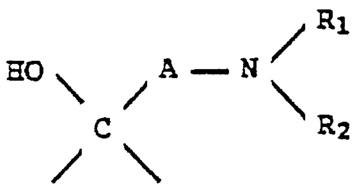 , 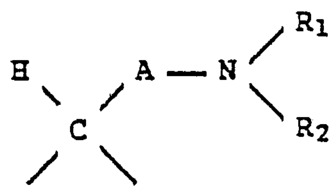 ;

A is a alkylene group of from 1 to 4 carbon atoms;

$R_1$ and $R_2$ are each, independently, a hydrogen or an alkyl group of from 1 to 4 carbon atoms or

$R_1$ and $R_2$ taken together with the nitrogen atom to which they are attached can form a N-piperazino- optionally substituted with a methyl, ethyl, or 2-hydroxyethyl group, N-piperidino-, N-morpholino-, or N-pyrrolidino radical;

R and R′ are each, independently, a hydrogen, hydroxy, bromo, chloro, iodo, or trifluoromethyl group or an alkyl or alkoxy group of from 1 to 4 carbon atoms;

or of a pharmaceutically acceptable salt thereof for the preparation of a pharmaceutical composition for treating or preventing a drug-resistant protozoal infection in a patient in conjunction with an antiprotozoal agent.

2. The use of claim 1 wherein Z is a 1,2-ethanediyl or a 1,2-ethenediyl group.

3. The use of claim 1 wherein Y is a group of the formula

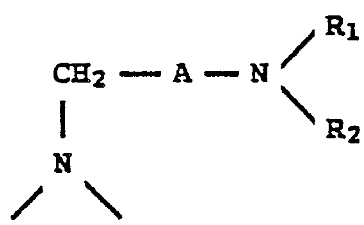

and Z is a 1,2-ethanediyl or 1,2-ethenediyl group.

4. The use of claim 3 wherein A is a 1,2-ethanediyl group and wherein R and R′ are each a hydrogen.

5. The use of claim 1 wherein Y is a group of the formula

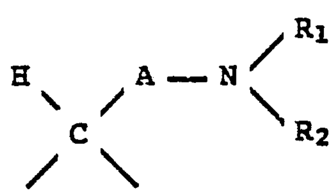

and Z is a 1,2-ethanediyl or 1,2-ethenediyl group.

6. The use of claim 1 wherein Y is a group of the formula

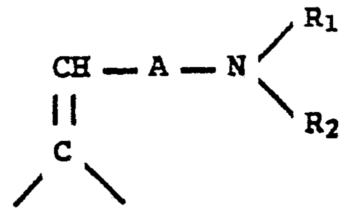

and Z is a 1,2-ethanediyl or 1,2 ethenediyl group.

7. The use of any one of claims 1,3,5 and 6 wherein at least one of $R_1$ and $R_2$ are an alkyl group of from 1 to 5 carbon atoms.

8. The use of any one of claims 1,3,5 and 6 wherein A is an alkylene group of from 1 to 3 carbon atoms.

9. The use of any one of claims 1,3,5 and 6 wherein R and $R'$ are each a hydrogen.

10. The use of any one of claims 1,3,5 and 6 wherein one of $R_1$ and $R_2$ is a methyl group and the other is a hydrogen or wherein both of $R_1$ and $R_2$ are methyl groups.

11. The use of any one of claims 1-10 wherein A is a 1,3-propanediyl group and wherein R and $R'$ are each a hydrogen.

12. The use of any one of claims 1-11 in which the drug-resistant protozoal infection is a drug-resistant malarial infection.

13. The use of claim 12 in which the antiprotozoal agent is quinine, chloroquine, amodiaquine, primaquine, or mefloquine.

14. An anti-drug-resistant protozoal pharmaceutical composition comprising a tricyclic compound of the formula I according to claim 1
or a pharmaceutically acceptable salt thereof and an antiprotozoal agent in amounts effective to treat or prevent the drug-resistant infection together with a pharmaceutically acceptable carrier.

15. A kit for use in the treatment or prevention of a drug-resistant protozoal infection which comprises
(a) a unit dosage form of a tricyclic compound of the formula I according to claim 1
or a pharmaceutically acceptable salt thereof together with a pharmaceutically acceptable carrier;
(b) a unit dosage form of an antiprotozoal agent together with a pharmaceutically acceptable carrier; and optionally
(c) printed instructions which describe the manner in which the unit dosage forms of the tricyclic compound and the antiprotozoal agent can be used to treat or prevent a drug-resistant protozoal infection.

16. A kit as in claim 15 wherein the tricyclic compound is desipramine and the antiprotozoal agent is chloroquine.

17. The use of a tricyclic compound of the formula I according to claim 1
or a pharmaceutically acceptable salt thereof in the preparation of pharmaceutical compositions intended for the treatment of drug-resistant protozoal infections.

FIGURE 1

DHI INHIBITION OF PLASMODIUM FALCIPARUM

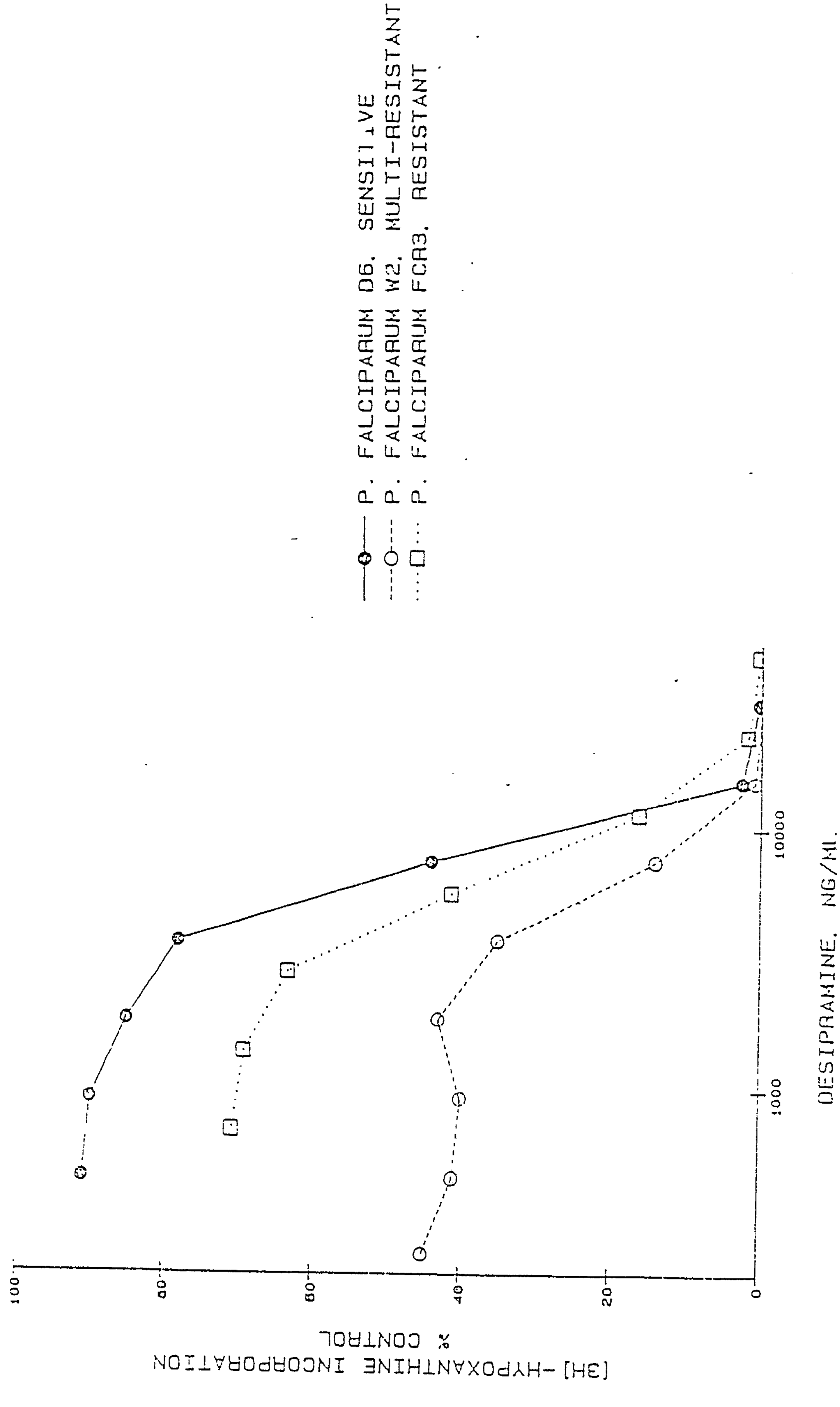

FIGURE 2

SYNERGISM OF DESIPRAMINE AND CHLOROQUINE AGAINST A CHLOROQUINE-RESISTANT P. FALCIPARUM

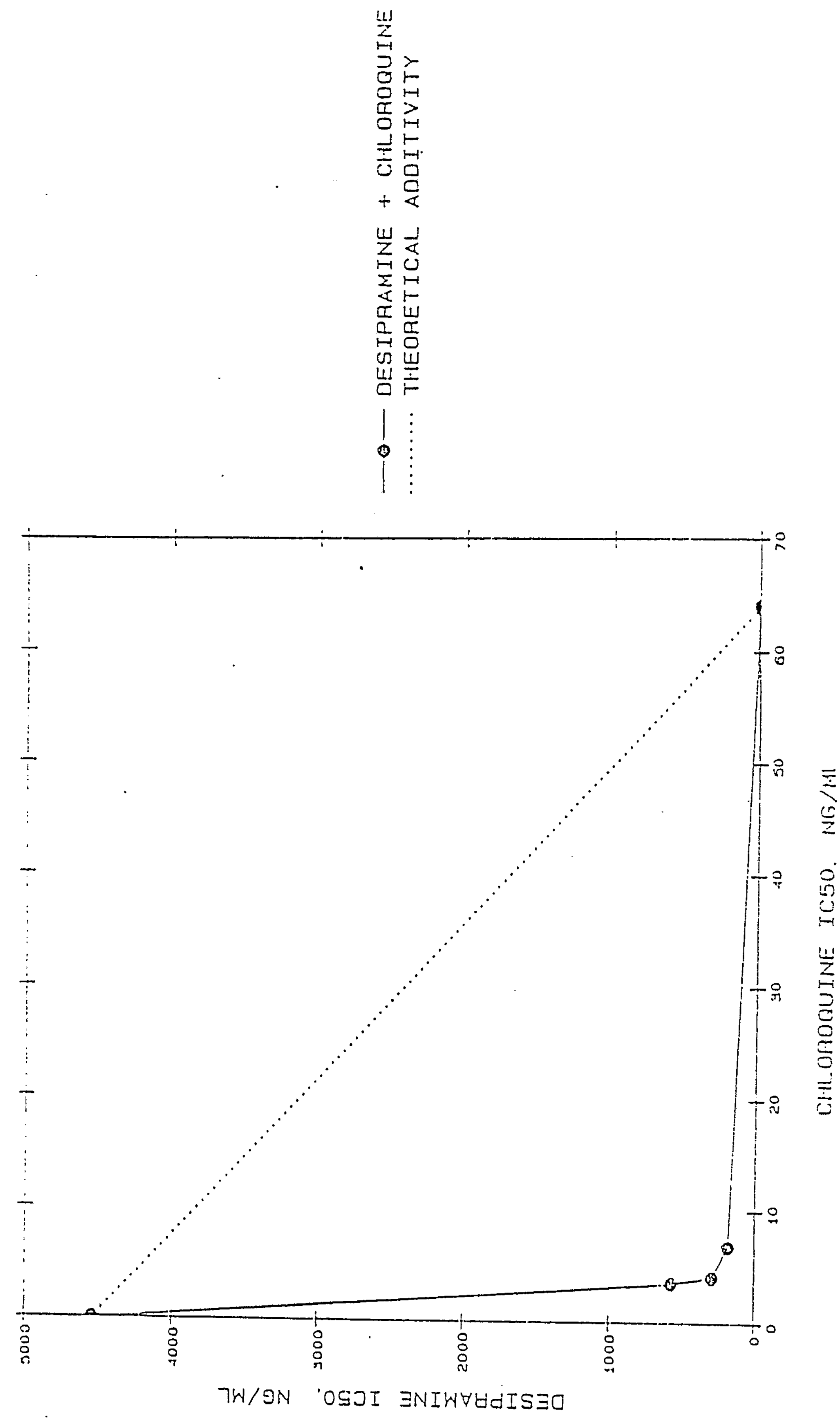

FIGURE 3

DMI + CHLOROQUINE AGAINST CHLOROQUINE-RESISTANT PLASMODIUM FALCIPARUM (FCR3)

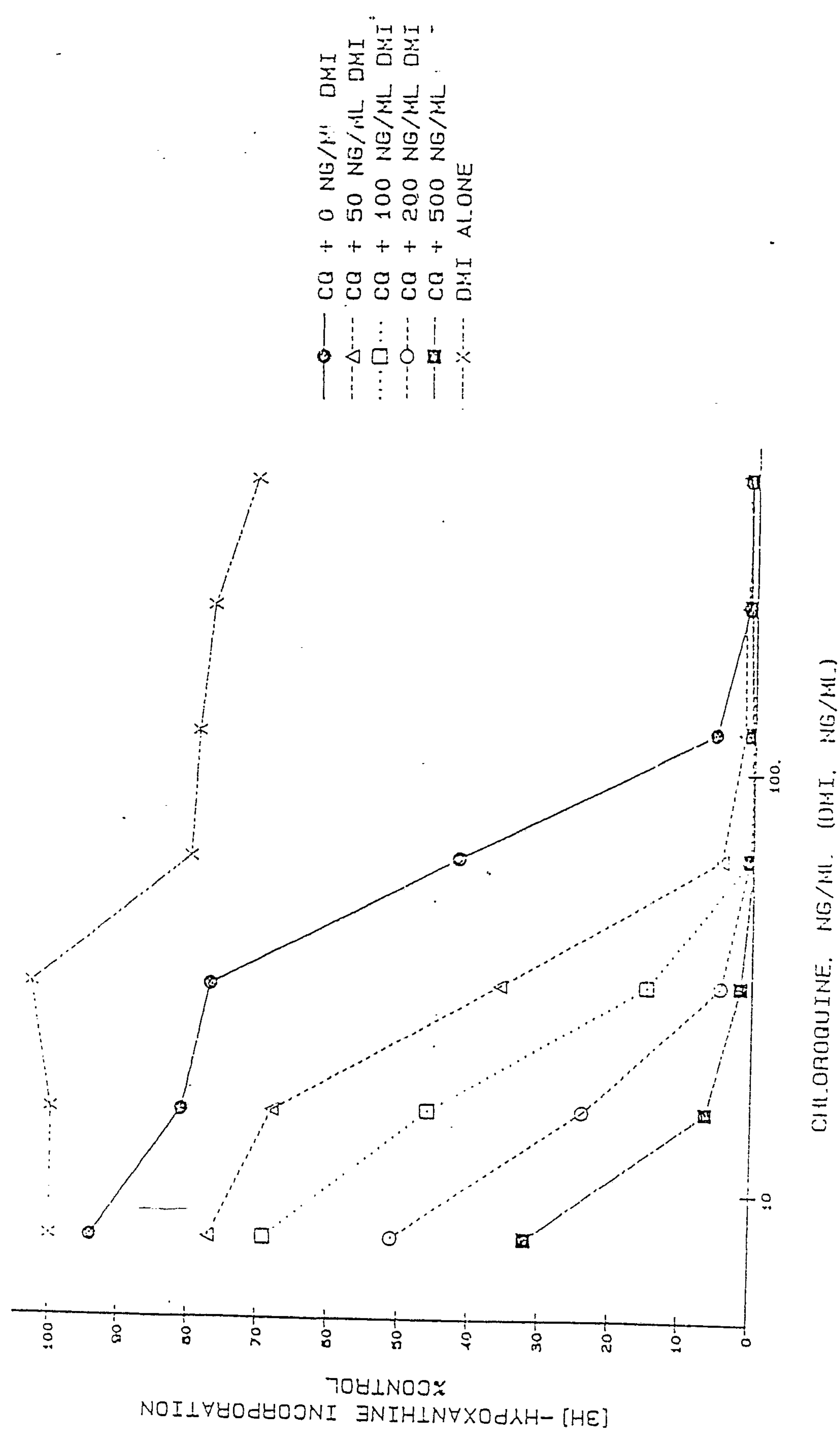

FIGURE 4

EFFECTS OF CHLOROQUINE + DMI ON A MULTI-DRUG RESISTANT PLASMODIUM FALCIPARUM (K2)

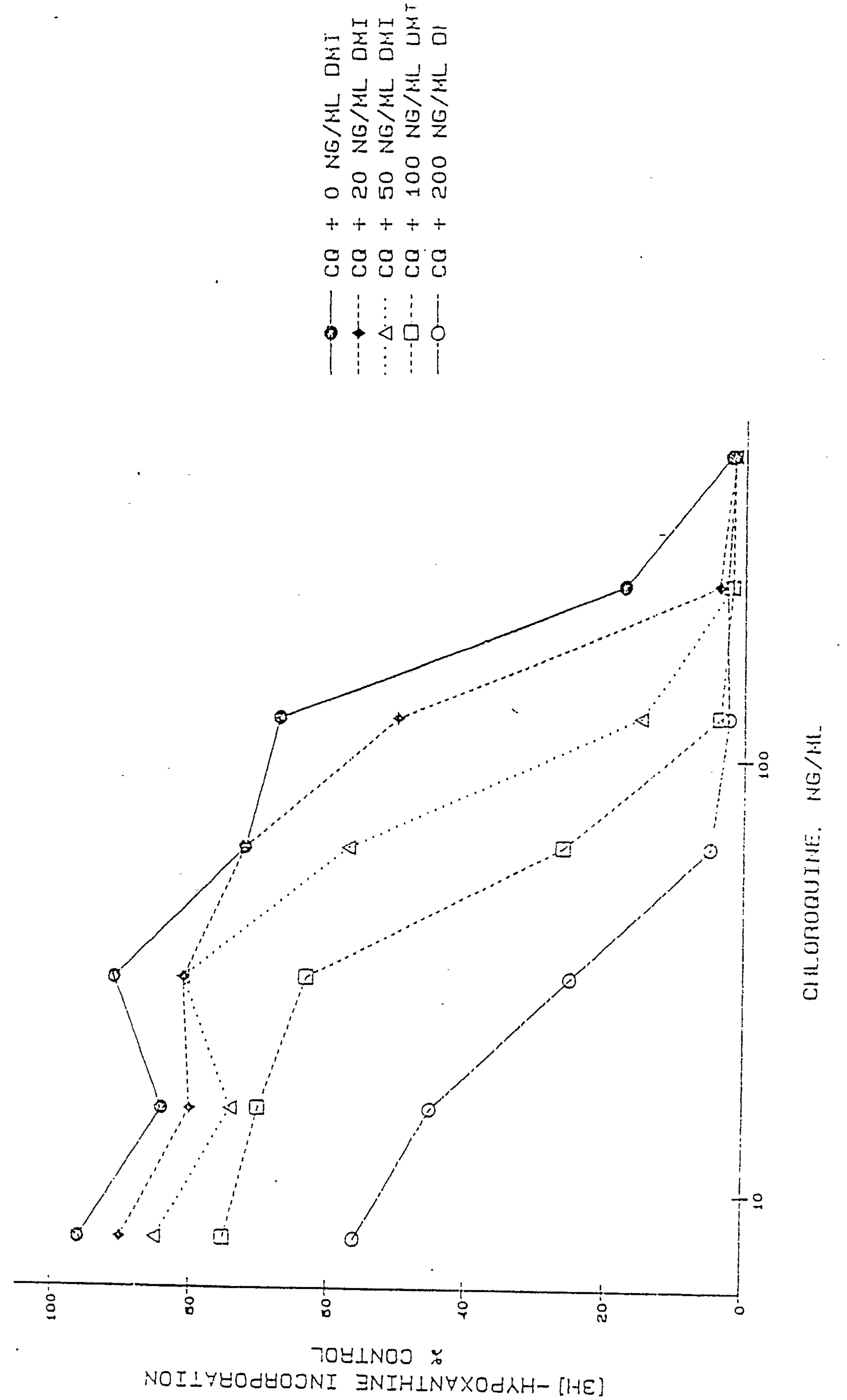

FIGURE 5

CHLOROQUINE PLUS OMI ON P. FALCIPARUM (D6)

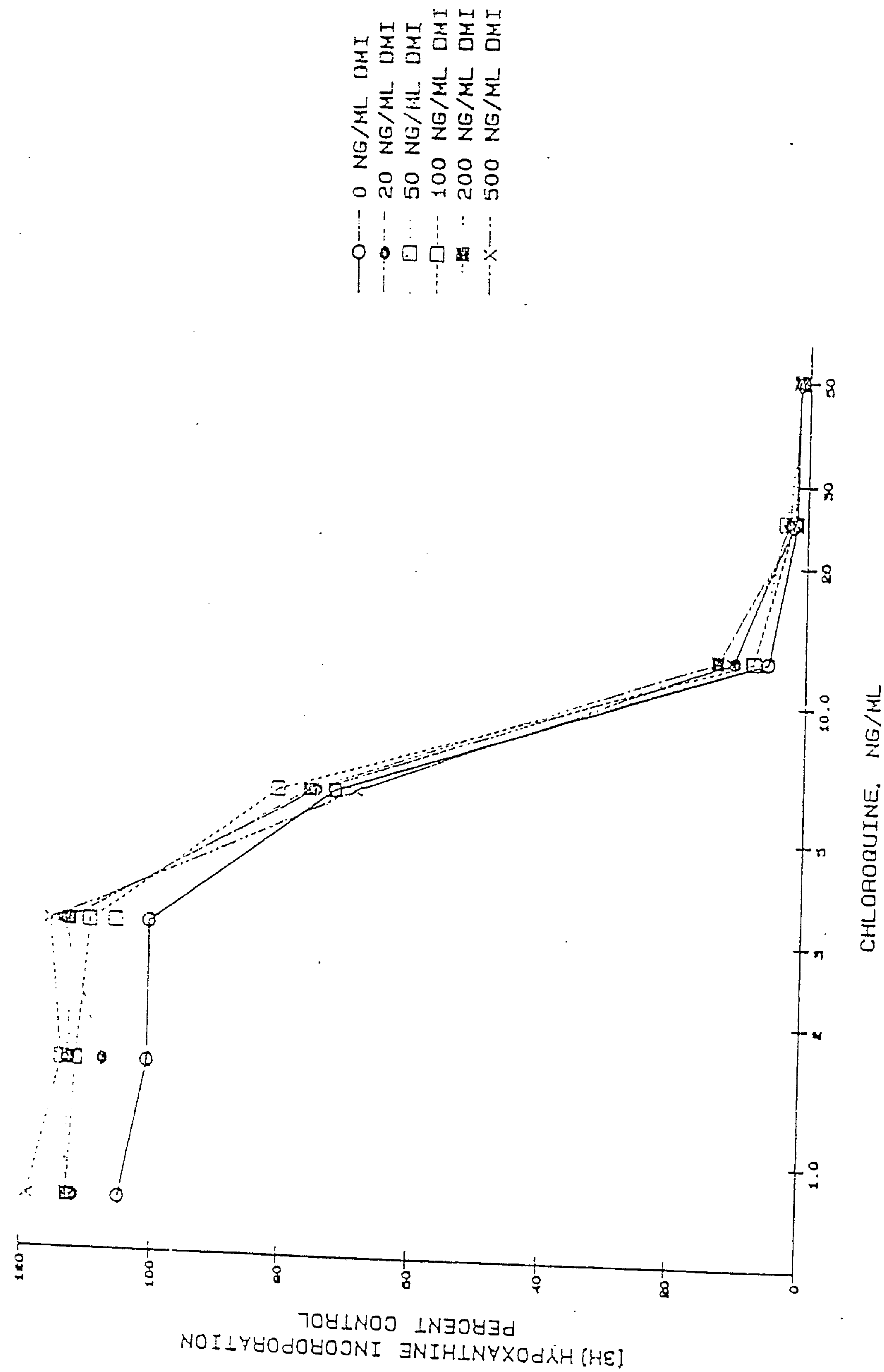

FIGURE 6

SYNERGISM BETWEEN CHLORPROMAZINE AND CHLOROQUINE AGAINST A CHLOROQUINE-RESISTANT PLASMODIUM FALCIPARUM (FCR3)

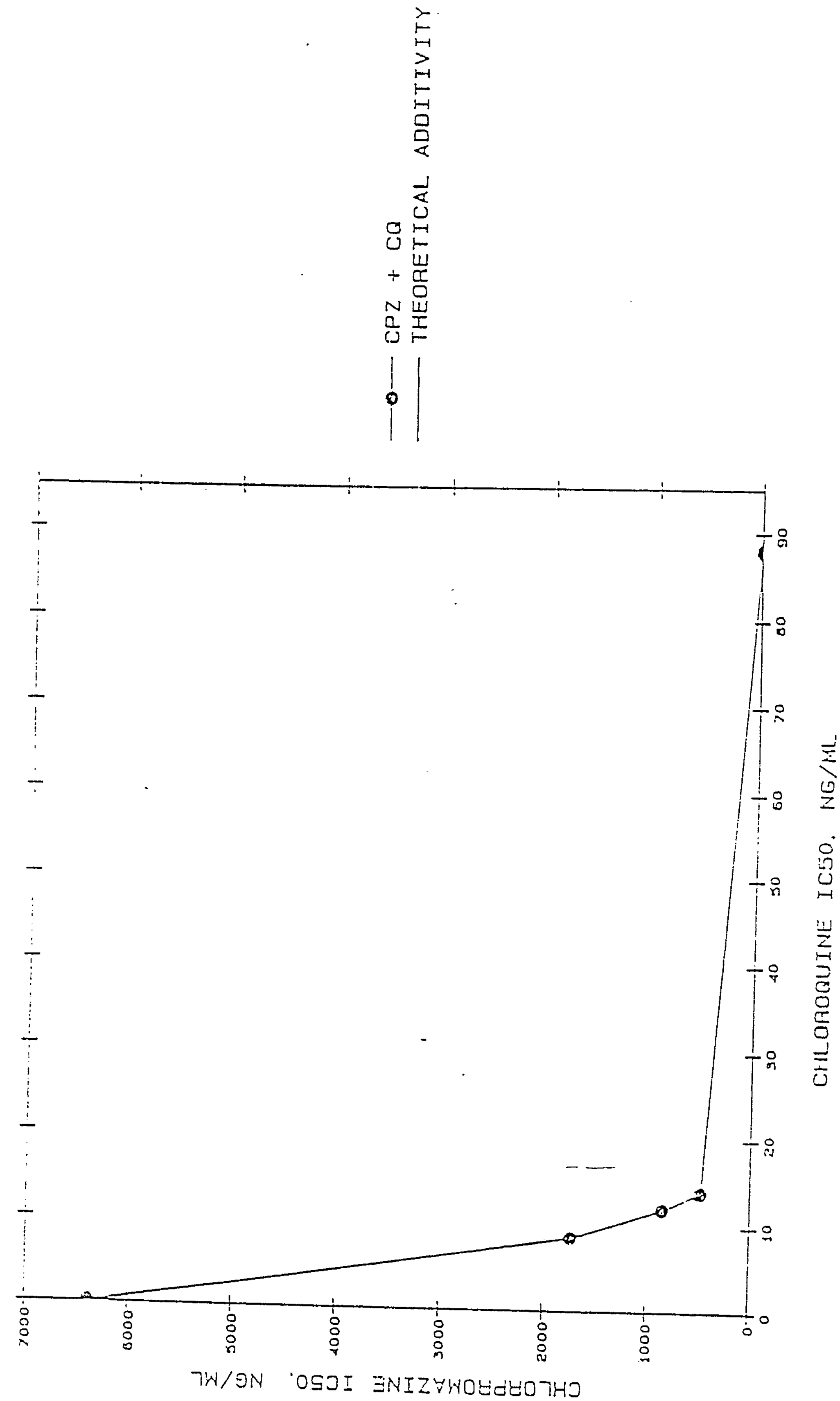

FIGURE 7

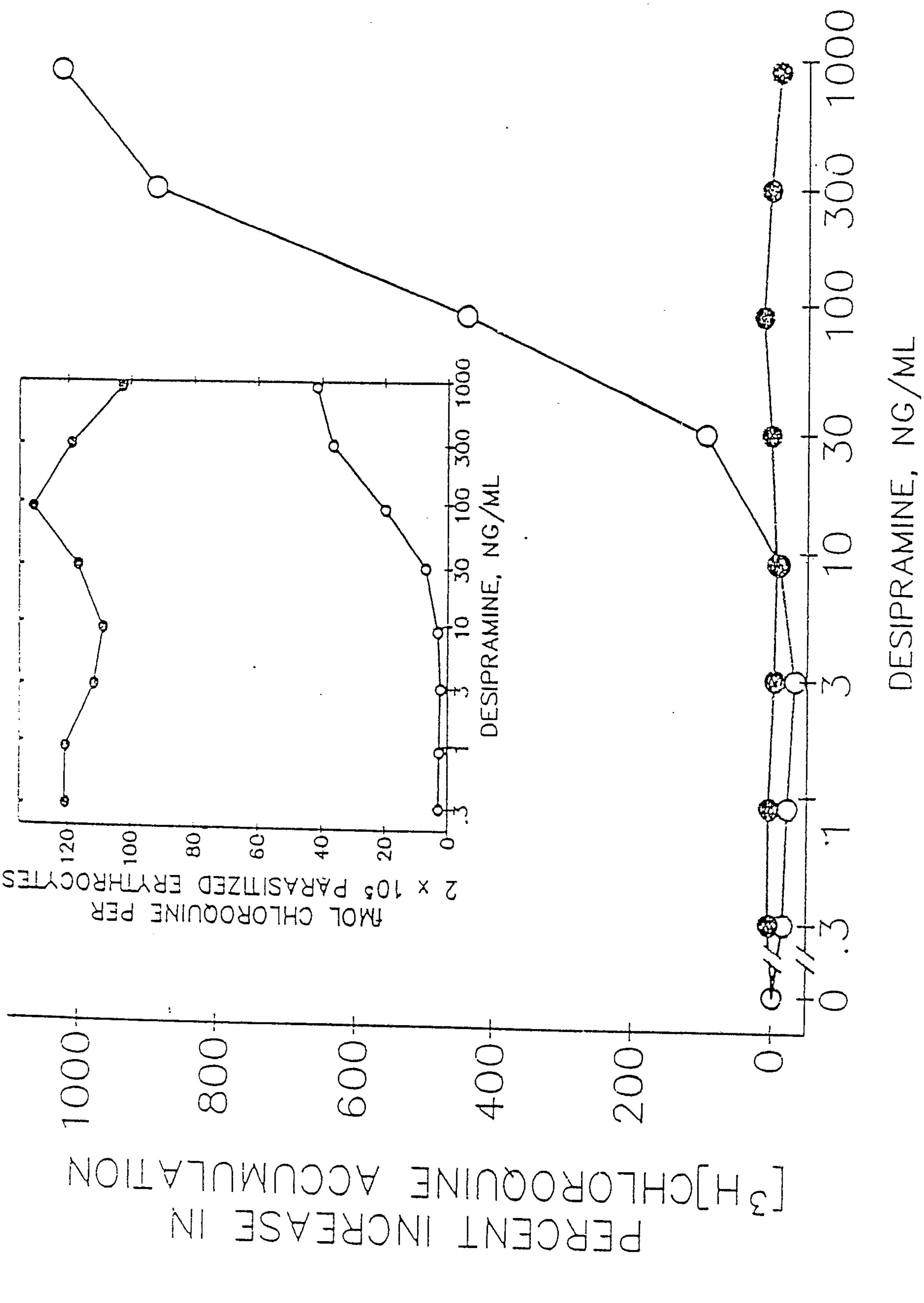
PERCENT INCREASE IN
[³H]CHLOROQUINE ACCUMULATION
0
200
400
600
800
1000
DESIPRAMINE, NG/ML
0
.3
1
3
10
30
100
300
1000
fMOL CHLOROQUINE PER
2 x 10⁵ PARASITIZED ERYTHROCYTES
0
20
40
60
80
100
120
DESIPRAMINE, NG/ML
.3
1
3
10
30
100
300
1000